# EUROPÄISCHE PATENTSCHRIFT

(11) **EP 0 605 769 B1**
(45) Veröffentlichungstag und Bekanntmachung des Hinweises auf die Patenterteilung: **29.01.1997**
(21) Anmeldenummer: 93118033.5
(22) Anmeldetag: 06.11.1993
(51) Int. Cl.: C07C 29/68, C07C 29/70, C07C 31/30, C08F 10/00, D21H 25/18

(54) **Lagerstabile Lösungen eines Gemisches aus carbonisiertem Magnesiummethylat, carbonisiertem Magnesiumethylat und deren carbonisiertem Mischalkoxid in einem Gemisch aus Methanol und Ethanol sowie deren Herstellung und Verwendung**
Storage stable solution of a mixture of carbonated magnesium methyloxide, carbonated magnesium ethoxide and their mixed alkoxides in a mixture of methanol and ethanol, their preparation and use
Solutions stables au stockage d'un mélange de méthylate de magnésium carbonaté, d'éthylate de magnésium carbonaté et leurs mélanges d'alkoxides carbonatés, dans un mélange de méthanol et d'éthanol, demême que leur préparation et utilisation

(30) Priorität: 07.01.1993 DE 4300185
(43) Veröffentlichungstag der Anmeldung: 13.07.1994
(73) Patentinhaber: HÜLS AKTIENGESELLSCHAFT, 45764 Marl (DE)
(72) Erfinder: Rauleder, Hartwig, Dr., D-79618 Rheinfelden (DE); Standke, Burkhard, Dr., D-79618 Rheinfelden (DE); Kötzsch, Hans-Joachim, Dr., D-79618 Rheinfelden (DE); Schork, Reinhold, Dr., D-79618 Rheinfelden (DE)

(56) Entgegenhaltungen:
- EP-A- 0 436 801
- EP-A- 0 491 128
- US-A- 4 318 963
- US-A- 4 540 679

## Beschreibung

Die Erfindung betrifft lagerstabile Lösungen eines Gemisches aus carbonisiertem Magnesiummethylat der Formel Mg(CH₃O)₂ (CO₂)ₙ, carbonisiertem Magnesiumethylat der Formel Mg(C₂H₅O)₂ (CO₂)ₙ und deren carbonisiertem Mischalkoxid der Formel Mg(CH₃O)ₓ (C₂H₅O)_{y} (CO₂)ₙ in einem Gemisch aus Methanol und Ethanol, wobei x und y größer als 0 und kleiner als 2 sind und die Summe aus x und y gleich 2 ist, sowie Verfahren zu deren Herstellung.

Außerdem betrifft die Erfindung die Verwendung der lagerstabilen Lösungen eines Gemisches aus carbonisiertem Magnesiummethylat, carbonisiertem Magnesiumethylat und deren carbonisiertem Mischalkoxid in einem Gemisch aus Methanol und Ethanol zur Konservierung von Papier sowie die Verwendung zur Herstellung von Katalysatoren für die Polymerisierung von Olefinen.

Magnesiumalkoholate sind in der Regel in den korrespondierenden Alkoholen wenig löslich bzw. nahezu unlöslich. Eine Ausnahme bildet Magnesiummethylat, das in Methanol eine Löslichkeit bis zu ca. 12 Gew-% aufweist.

Eine technisch einfache Methode, die Löslichkeit von Magnesiumalkoholaten zu erhöhen, stellt die Carbonisierung dar. In eine Suspension des Magnesiumalkoholates im korrespondierenden Alkohol wird gasförmiges, flüssiges oder festes CO₂ eingebracht. Hierbei bildet sich ein lösliches CO₂-Addukt. Das CO₂-Addukt ist im Falle von Magnesiumethylat in Ethanol mit einer Konzentration von über 30 Gew.-% löslich, hingegen ist reines Magnesiumethylat in Ethanol nahezu unlöslich.

Alkohollösliche carbonisierte Magnesiumalkoxide finden vielfache Anwendung. So können beispielsweise durch Carbonisierung in Lösung gebrachte Magnesiumalkoxide bei der Langzeitkonservierung von Papier, insbesondere von Büchern, verwendet werden und die zu diesem Zweck gemäß US-PS 3 969 549, EP-A 0 285 227, US-PS 4 318 963 und US-PS 3 939 091 ebenfalls eingesetzten, in der Anwendung jedoch problematischen, Zinkalkyle substituieren.

Ein weiteres Anwendungsfeld ist gemäß EP-PS 0 159 150 und EP-PS 0 236 082 die Herstellung von Katalysatoren für die Polymerisierung von Olefinen. Hierfür benötigt man z. B. sphärisches Magnesiummethylat oder Magnesiumethylat, das beispielsweise durch Sprühtrocknung oder Fällung carbonisierter Magnesiummethylatlösungen oder Magnesiumethylatlösungen und gegebenenfalls anschließende Decarbonisierung hergestellt werden kann.

Es ist prinzipiell bekannt, daß Lösungen carbonisierter Magnesiumalkoholate nach den beiden folgenden Reaktionsschemata hergestellt werden können, wobei einmal metallisches Magnesium mit einem Alkohol ROH und CO₂ (siehe Reaktionsgleichung 1) und zum anderen ein Magnesiumalkoxid mit CO₂ (siehe Reaktionsgleichung 2) umgesetzt werden:

Mg + 2 ROH + n CO₂ → Mg(OR)₂ (CO₂)ₙ + H₂ (1)

Mg(OR)₂ + n CO₂ → Mg(OR)₂ (CO₂)ₙ (2)

Hierbei bedeutet R eine Alkylgruppe.

So lehrt EP-PS 0 236 082 die Herstellung von carbonisierten Magnesiumalkoxiden durch Reaktion von Magnesiumalkoxiden mit CO₂ in einem Lösungsmittel. Bevorzugte Lösungsmittel sind Alkohole. Beispielsweise wird auch die Herstellung von carbonisiertem Magnesiumethylat durch Umsetzung von Magnesiumethylat mit CO₂ in Ethanol als Lösungsmittel offenbart.

Fieser und Fieser beschreiben in "Reagents for Organic Synthesis", Vol. 1, Seite 631, die Herstellung von carbonisiertem Magnesiummethylat auf zwei verschiedenen Wegen:
Zum einen wird eine Suspension von Magnesiummethylat in Methanol mit CO₂ umgesetzt.
Zum anderen läßt man Magnesiumspäne mit Methanol zu Magnesiummethylat reagieren. Nachdem der Methanol teilweise bei 50 °C und Unterdruck abgezogen worden ist, wird Dimethylformamid als Lösungsmittel zugesetzt und CO₂ in diese Lösung eingeleitet. Der restliche Methanol wird abdestilliert, und man erhält eine schwach gelbe Lösung von carbonisiertem Magnesiummethylat in Dimethylformamid.

In EP-PS 0 159 150 wird über die Verwendung von Lösungen carbonisierter Magnesiumalkoholate in Alkoholen zur Herstellung aktiver Katalysatorträgermaterialien für die Olefinpolymerisation berichtet. Die verwendeten Lösungen enthalten 10 bis 80 Gew.-% Magnesiumalkoholat, 0,1 bis 4 mol CO₂ pro mol Magnesium und werden durch Umsetzung von Magnesiumalkoholat, dispergiert in Alkohol, mit gasförmigem CO₂ hergestellt.

Die alkoholischen Lösungen carbonisierter Magnesiumalkoxide gemäß dem Stand der Technik sind jedoch gelb bis rot gefärbt, wobei sich die Verfärbung mit zunehmender Lagerdauer verstärken kann. Hinzu kommt, daß bei Lagerung selbst in dicht verschlossenen Gefäßen schon nach einigen Tagen Ausfällungen oder Gelierungen auftreten können. Die technische Verwendung solcher Lösungen ist erheblich eingeschränkt: Einerseits können verfärbte Lösungen beispielsweise nicht für die Langzeitkonservierung von Büchern verwendet werden, andererseits sind Lösungen, bei denen die Gefahr unkontrollierter Ausfällungen besteht, für die Herstellung von Katalysatoren auf Basis Magnesiumalkoxid unbrauchbar.

Der Erfindung liegt die Aufgabe zugrunde, lagerstabile Lösungen von carbonisierten Magnesiumalkoxiden herzustellen, die auch über einen längeren Zeitraum nahezu farblos bleiben und weder Ausfällungen noch Gelierung zeigen.

Es wurde nun überraschenderweise gefunden, daß durch Einstellung definierter Magnesium- und CO₂-Konzentrationen in den erfindungsgemäßen Lösungen eines Gemisches aus carbonisiertem Magnesiummethylat, carbonisiertem Magnesiumethylat und deren carbonisiertem Mischalkoxid in einem Gemisch aus Methanol und Ethanol Verfärbungen, Ausfällungen und Gelierungen auch während der Lagerung über lange Zeiträume nicht auftreten.

Gegenstand der vorliegenden Erfindung sind daher lagerstabile Lösungen eines Gemisches aus carbonisiertem Magnesiummethylat der Formel Mg(CH₃O)₂ (CO₂)ₙ, carbonisiertem Magnesiumethylat der Formel Mg(C₂H₅O)₂ (CO₂)ₙ und deren carbonisiertem Mischalkoxid der Formel Mg(CH₃O)ₓ (C₂H₅O)_{y} (CO₂)ₙ in einem Gemisch aus Methanol und Ethanol, wobei x und y größer als 0 und kleiner als 2 sind und die Summe aus x und y gleich 2 ist, die dadurch gekennzeichnet sind, daß der Magnesiumgehalt der Lösung 0,1 bis 11 Gew.-%, bezogen auf die gesamte Lösung, und der CO₂-Gehalt n gleich 1,2 bis 2,2 betragen.

Des weiteren ist Gegenstand der vorliegenden Erfindung ein Verfahren zur Herstellung lagerstabiler Lösungen eines Gemisches aus carbonisiertem Magnesiummethylat der Formel Mg(CH₃O)₂ (CO₂)ₙ, carbonisiertem Magnesiumethylat der Formel Mg(C₂H₅O)₂ (CO₂)ₙ und deren carbonisiertem Mischalkoxid der Formel Mg(CH₃O)ₓ (C₂H₅O)_{y} (CO₂)ₙ in einem Gemisch aus Methanol und Ethanol, wobei x und y größer als 0 und kleiner als 2 sind und die Summe aus x und y gleich 2 ist, das dadurch gekennzeichnet ist, daß Magnesiumethylat in Methanol oder Magnesiumethylat und Magnesiummethylat in Methanol oder Magnesiummethylat in Ethanol oder Magnesiummethylat und Magnesiumethylat in Ethanol und/oder deren Mischalkoxid der Formel Mg(CH₃O)ₓ (C₂H₅O)_{y}, wobei x und y größer als 0 und kleiner als 2 sind und die Summe aus x und y gleich 2 ist, in Ethanol oder Methanol mit CO₂ umgesetzt und der Magnesiumgehalt der Lösung auf Werte von 0,1 bis 11 Gew.-%, bezogen auf die gesamte Lösung, und der CO₂-Gehalt auf Werte von n gleich 1,2 bis 2,2 eingestellt werden sowie ein
Verfahren zur Herstellung lagerstabiler Lösungen eines Gemisches aus carbonisiertem Magnesiummethylat der Formel Mg(CH₃O)₂ (CO₂)ₙ, carbonisiertem Magnesiumethylat der Formel Mg(C₂H₅O)₂ (CO₂)ₙ und deren carbonisiertem Mischalkoxid der Formel Mg(CH₃O)ₓ (C₂H₅O)_{y} (CO₂)ₙ in einem Gemisch aus Methanol und Ethanol, wobei x und y größer als 0 und kleiner als 2 sind und die Summe aus x und y gleich 2 ist, das dadurch gekennzeichnet ist, daß Magnesiumethylat und/oder Magnesiummethylat und/oder deren Mischalkoxid der Formel Mg(CH₃O)ₓ (C₂H₅O)_{y}, wobei x und y größer als 0 und kleiner als 2 sind und die Summe aus x und y gleich 2 ist, in einem Gemisch aus Methanol und Ethanol mit CO₂ umgesetzt und der Magnesiumgehalt der Lösung auf Werte von 0,1 bis 11 Gew.-%, bezogen auf die gesamte Lösung, und der CO₂-Gehalt auf Werte von n gleich 1,2 bis 2,2 eingestellt werden.

Außerdem ist Gegenstand der vorliegenden Erfindung ein Verfahren zur Herstellung lagerstabiler Lösungen eines Gemisches aus carbonisiertem Magnesiummethylat der Formel Mg(CH₃O)₂ (CO₂)ₙ, carbonisiertem Magnesiumethylat der Formel Mg(C₂H₅O)₂ (CO₂)ₙ und deren carbonisiertem Mischalkoxid der Formel Mg(CH₃O)ₓ (C₂H₅O)_{y} (CO₂)ₙ in einem Gemisch aus Methanol und Ethanol, wobei x und y größer als 0 und kleiner als 2 sind und die Summe aus x und y gleich 2 ist, das dadurch gekennzeichnet ist, daß metallisches Magnesium mit einem Gemisch aus Methanol und Ethanol sowie CO₂ umgesetzt wird und der Magnesiumgehalt der Lösung auf Werte von 0,1 bis 11 Gew.-%, bezogen auf die gesamte Lösung, und der CO₂-Gehalt auf Werte von n gleich 1,2 bis 2,2 eingestellt werden.

Darüber hinaus ist Gegenstand der vorliegenden Erfindung die Verwendung der lagerstabilen Lösungen eines Gemisches aus carbonisiertem Magnesiummethylat, carbonisiertem Magnesiumethylat und deren carbonisiertem Mischalkoxid in einem Gemisch aus Methanol und Ethanol nach Anspruch 1 zur Konservierung von Papier sowie die Verwendung der lagerstabilen Lösungen eines Gemisches aus carbonisiertem Magnesiummethylat, carbonisiertem Magnesiumethylat und deren carbonisiertem Mischalkoxid in einem Gemisch aus Methanol und Ethanol nach Anspruch 1 zur Herstellung von Katalysatoren für die Polymerisierung von Olefinen.

Bei der Herstellung der erfindungsgemäßen Lösungen dient als Magnesiumquelle entweder metallisches Magnesium (siehe Gleichung 1) oder Magnesiumethylat und/oder Magnesiummethylat und/oder deren Mischalkoxid (siehe Gleichung 2).

Die Verwendung von metallischem Magnesium ist wirtschaftlich vorteilhafter, da z. B. Magnesiumethylat üblicherweise aus Magnesiummetall durch Umsetzung mit Ethanol gewonnen wird und somit ein Reaktionsschritt entfällt. Weiterhin lassen sich bei der Umsetzung von metallischem Magnesium mit einem Gemisch aus Methanol und Ethanol sowie CO₂ bezüglich der Farbqualität hochwertigere Produkte erzeugen. Man erhält also bei der Umsetzung von metallischem Magnesium mit einem Gemisch aus Methanol und Ethanol sowie CO₂ erfindungsgemäße Lösungen, die im Vergleich mit den z. B. aus Magnesiumethylat in Methanol oder Magnesiummethylat in Ethanol mit CO₂ hergestellten erfindungsgemäßen Lösungen etwas geringere Farbzahlen, gemessen nach der Meßmethode von Gardner, aufweisen.

Aufgrund der Tatsache, daß bei der Umsetzung von metallischem Magnesium mit einem Gemisch aus Methanol und Ethanol sowie CO₂ die Oberfläche des Magnesiums wegen der Löslichkeit des carbonisierten Magnesiummethylats, carbonisiertem Magnesiumethylats und deren carbonisierten Mischalkoxids ständig freiliegt, erfolgt keine Passivierung der Metalloberfläche durch die Bildung einer unslöslichen Grenzschicht zwischen Metall und Methanol und/oder Ethanol. Man ist daher bei der Herstellung der erfindungsgemäßen Lösungen aus metallischem Magnesium mit einem Gemisch aus Methanol und Ethanol sowie CO₂ nicht auf die Verwendung von oberflächenreichem Magnesiummaterial, wie z. B. Magnesiumspänen oder Magnesiumgrieß, angewiesen, wie es beispielsweise bei der technischen Herstellung von Magnesiumethylat oder Magnesiummethylat benötigt wird, sondern kann preislich günstigere und sicherheitstechnisch unproblematischer zu handhabende Magnesiumblockware einsetzen. Weiterhin benötigt man zum Starten der Reaktion keinerlei Hilfssubstanzen, wie beispielsweise Quecksilbersalze (siehe Liebigs Annalen der Chemie 444, 236, 1925), so daß die erreichbare Produktreinheit bei Einsatz von Magnesiummetall im Vergleich mit der bei Einsatz von Magnesiumethylat und/oder Magnesiummethylat und/oder deren Mischalkoxid etwas größer ist.

Die Carbonisierung kann durch Einleitung von gasförmigem CO₂ oder durch Zugabe von flüssigem oder festem CO₂ in das Reaktionsgemisch aus metallischem Magnesium und einem Gemisch aus Methanol und Ethanol
bzw. aus Magnesiumethylat und Methanol oder
Magnesiumethylat und Magnesiummethylat und Methanol oder
Magnesiummethylat und Ethanol oder
Magnesiummethylat und Magnesiumethylat und Ethanol und/oder
deren Mischalkoxid der Formel Mg(CH₃O)ₓ (C₂H₅O)_{y}, wobei x und y größer als 0 und kleiner als 2 sind und die Summe aus x und y gleich 2 ist und Ethanol oder Methanol
oder
aus Magnesiumethylat und/oder Magnesiummethylat und/oder deren Mischalkoxid der Formel Mg(CH₃O)ₓ (C₂H₅O)_{y}, wobei x und y größer als 0 und kleiner als 2 sind und die Summe aus x und y gleich 2 ist, und einem Gemisch aus Methanol und Ethanol erfolgen.

Es kann also der CO₂-Gehalt n der erfindungsgemäßen Lösung durch Zudosieren von gasförmigem, flüssigem oder festem CO₂ in das Gemisch eingestellt werden.

Eine andere Variante ist, daß der CO₂-Gehalt n der erfindungsgemäßen Lösung durch thermisches Austreiben von CO₂ aus einer einen Überschuß an CO₂ enthaltenden Lösung eines Gemisches aus carbonisiertem Magnesiummethylat, carbonisiertem Magnesiumethylat und deren carbonisiertem Mischalkoxid in einem Gemisch aus Methanol und Ethanol eingestellt werden kann.

Die Menge des eingebrachten CO₂ ist einfach über Massenbilanzierung durch Gewichtszunahme kontrollierbar. Der Magnesiumgehalt der erfindungsgemäßen Lösung kann durch destillative Entfernung von Methanol und/oder Ethanol aus einer einen Überschuß an Methanol und/oder Ethanol enthaltenden Lösung eines Gemisches aus carbonisiertem Magnesiummethylat, carbonisiertem Magnesiumethylat und deren carbonisiertem Mischalkoxid in einem Gemisch aus Methanol und Ethanol eingestellt werden.

Aufgrund der Hydrolyse- und Oxidationsempfindlichkeit der erfindungsgemäßen Lösungen sind sämtliche Operationen unter Ausschluß von Luft und Feuchtigkeit durchzuführen.

Die Erfindung wird durch die folgenden Beispiele näher erläutert:

### Beispiel 1

Herstellung einer lagerstabilen Lösung eines Gemisches aus carbonisiertem Magnesiummethylat, carbonisiertem Magnesiumethylat und deren carbonisiertem Mischalkoxid durch Umsetzung von Magnesiumethylat in Methanol mit CO₂.

In einem Dreihalskolben, ausgerüstet mit Rückflußkühler, Gaseinleitungsrohr, Stickstoffüberlagerung und KPG-Flügelrührer, werden 470 g Methanol vorgelegt und anschließend 200 g Magnesiumethylat (Hersteller: Hüls AG) unter Stickstoffbeschleierung zugesetzt. Unter Rühren wird CO₂ eingeleitet. Die CO₂-Aufnahme wird über Gewichtskontrolle kontinuierlich verfolgt. Die Temperatur im Reaktionsraum steigt kontinuierlich an. Bei Aufnahme von 92 g CO₂, entsprechend einem CO₂-Gehalt n gleich 1,12, wird die Reaktion beendet. Die nahezu farblose Lösung mit einer Farbzahl von < 1 (gemessen nach "Gardner") besitzt einen Magnesiumgehalt von 6 Gew.-%. Nach 6 Wochen Lagerung in dichtverschlossener Glasflasche ist die Lösung unverändert.

Es tritt weder eine Verfärbung noch Ausfällung oder Gelierung auf.

### Beispiel 2

Herstellung einer lagerstabilen Lösung eines Gemisches aus carbonisiertem Magnesiummethylat, carbonisiertem Magnesiumethylat und carbonisiertem Mischalkoxid mit hoher Magnesiumkonzentration.

In einem Dreihalskolben, ausgerüstet mit Rückflußkühler, Gaseinleitungsrohr, Stickstoffüberlagerung und KPG-Flügelrührer, werden 470 g Methanol vorgelegt und anschließend 200 g Magnesiumethylat (Hersteller: Hüls AG) unter Stickstoffbeschleierung zugesetzt. Unter Rühren wird CO₂ eingeleitet. Die CO₂-Aufnahme wird über Gewichtskontrolle kontinuierlich verfolgt. Die Temperatur im Reaktionsraum steigt kontinuierlich an. Bei Aufnahme von 135 g CO₂, entsprechend einem CO₂-Gehalt n gleich 1,75, wird die Reaktion beendet. Die nahezu farblose Lösung besitzt einen Magnesiumgehalt von 5,7 Gew.-%.

Zur Einstellung einer höheren Magnesiumkonzentration werden 500 g der Lösung am Rotationsverdampfer bei einer von 20 auf 40 °C steigenden Sumpftemperatur und bei einem von 220 auf 10 mbar absolut sinkenden Druck in einem Zeitraum von ca. 3 Stunden eingeengt. Die Lösung verliert hierbei ca. 26 g CO₂ und 215 g Lösungsmittel (Gemisch aus Methanol und Ethanol). Der CO₂-Gehalt n der Lösung beträgt 1,2, der Magnesiumgehalt 11 Gew.-% und die Farbzahl < 1 (gemessen nach "Gardner"). Nach 6 Wochen Lagerung in dicht verschlossener Glasflasche ist die Lösung unverändert. Es tritt weder eine Verfärbung noch Ausfällung oder Gelierung auf.

## Patentansprüche

1. Lagerstabile Lösungen eines Gemisches aus carbonisiertem Magnesiummethylat der Formel Mg(CH₃O)₂ (CO₂)ₙ, carbonisiertem Magnesiumethylat der Formel Mg(C₂H₅O)₂ (CO₂)ₙ und deren carbonisiertem Mischalkoxid der Formel Mg(CH₃O)ₓ (C₂H₅O)_{y} (CO₂)ₙ in einem Gemisch aus Methanol und Ethanol, wobei x und y größer als 0 und kleiner als 2 sind und die Summe aus x und y gleich 2 ist,
dadurch gekennzeichnet,
daß der Magnesiumgehalt der Lösung 0,1 bis 11 Gew.-%, bezogen auf die gesamte Lösung, und der CO₂-Gehalt n gleich 1,2 bis 2,2 betragen.

2. Verfahren zur Herstellung lagerstabiler Lösungen eines Gemisches aus carbonisiertem Magnesiummethylat der Formel Mg(CH₃O)₂ (CO₂)ₙ, carbonisiertem Magnesiumethylat der Formel Mg(C₂H₅O)₂ (CO₂)ₙ und deren carbonisiertem Mischalkoxid der Formel Mg(CH₃O)ₓ (C₂H₅O)_{y} (CO₂)ₙ in einem Gemisch aus Methanol und Ethanol, wobei x und y größer als 0 und kleiner als 2 sind und die Summe aus x und y gleich 2 ist, nach Anspruch 1,
dadurch gekennzeichnet,
daß Magnesiumethylat in Methanol oder Magnesiumethylat und Magnesiummethylat in Methanol oder Magnesiummethylat in Ethanol oder Magnesiummethylat und Magnesiumethylat in Ethanol und/oder deren Mischalkoxid der Formel Mg(CH₃O)ₓ (C₂H₅O)_{y}, wobei x und y größer als 0 und kleiner als 2 sind und die Summe aus x und y gleich 2 ist, in Ethanol oder Methanol mit CO₂ umgesetzt und der Magnesiumgehalt der Lösung auf Werte von 0,1 bis 11 Gew.-%, bezogen auf die gesamte Lösung, und der CO₂-Gehalt auf Werte von n gleich 1,2 bis 2,2 eingestellt werden.

3. Verfahren zur Herstellung lagerstabiler Lösungen eines Gemisches aus carbonisiertem Magnesiummethylat der Formel Mg(CH₃O)₂ (CO₂)ₙ, carbonisiertem Magnesiumethylat der Formel Mg(C₂H₅O)₂ (CO₂)ₙ und deren carbonisiertem Mischalkoxid der Formel Mg(CH₃O)ₓ (C₂H₅O)_{y} (CO₂)ₙ in einem Gemisch aus Methanol und Ethanol, wobei x und y größer als 0 und kleiner als 2 sind und die Summe aus x und y gleich 2 ist, nach Anspruch 1,
dadurch gekennzeichnet,
daß Magnesiumethylat und/oder Magnesiummethylat und/oder deren Mischalkoxid der Formel Mg(CH₃O)ₓ (C₂H₅O)_{y}, wobei x und y größer als 0 und kleiner als 2 sind und die Summe aus x und y gleich 2 ist, in einem Gemisch aus Methanol und Ethanol mit CO₂ umgesetzt und der Magnesiumgehalt der Lösung auf Werte von 0,1 bis 11 Gew.-%, bezogen auf die gesamte Lösung, und der CO₂-Gehalt auf Werte von n gleich 1,2 bis 2,2 eingestellt werden.

4. Verfahren zur Herstellung lagerstabiler Lösungen eines Gemisches aus carbonisiertem Magnesiummethylat der Formel Mg(CH₃O)₂ (CO₂)ₙ, carbonisiertem Magnesiumethylat der Formel Mg(C₂H₅O)₂ (CO₂)ₙ und deren carbonisiertem Mischalkoxid der Formel Mg(CH₃O)ₓ (C₂H₅O)_{y} (CO₂)ₙ in einem Gemisch aus Methanol und Ethanol, wobei x und y größer als 0 und kleiner als 2 sind und die Summe aus x und y gleich 2 ist, nach Anspruch 1,
dadurch gekennzeichnet,
daß metallisches Magnesium mit einem Gemisch aus Methanol und Ethanol sowie CO₂ umgesetzt wird und der Magnesiumgehalt der Lösung auf Werte von 0,1 bis 11 Gew.-%, bezogen auf die gesamte Lösung, und der CO₂-Gehalt auf Werte von n gleich 1,2 bis 2,2 eingestellt werden.

5. Verfahren nach den Ansprüchen 2 bis 4,
dadurch gekennzeichnet,
daß der CO₂-Gehalt n der Lösung durch Zudosieren von gasförmigem, flüssigem oder festem CO₂ in das Gemisch eingestellt wird.

6. Verfahren nach den Ansprüchen 2 bis 4,
dadurch gekennzeichnet,
daß der CO₂-Gehalt n der Lösung durch thermisches Austreiben von CO₂ aus einer einen Überschuß an CO₂ enthaltenden Lösung eines Gemisches aus carbonisiertem Magnesiummethylat, carbonisiertem Magnesiumethylat und deren carbonisiertem Mischalkoxid in einem Gemisch aus Methanol und Ethanol eingestellt wird.

7. Verfahren nach den Ansprüchen 2 bis 4,
dadurch gekennzeichnet,
daß der Magnesiumgehalt der Lösung durch destillative Entfernung von Methanol und/oder Ethanol aus einer einen Überschuß an Methanol und/oder Ethanol enthaltenden Lösung eines Gemisches aus carbonisiertem Magnesiummethylat, carbonisiertem Magnesiumethylat und deren carbonisiertem Mischalkoxid in einem Gemisch aus Methanol und Ethanol eingestellt wird.

8. Verwendung der lagerstabilen Lösungen eines Gemisches aus carbonisiertem Magnesiummethylat, carbonisiertem Magnesiumethylat und deren carbonisiertem Mischalkoxid in einem Gemisch aus Methanol und Ethanol nach Anspruch 1 zur Konservierung von Papier.

9. Verwendung der lagerstabilen Lösungen eines Gemisches aus carbonisiertem Magnesiummethylat, carbonisiertem Magnesiumethylat und deren carbonisiertem Mischalkoxid in einem Gemisch aus Methanol und Ethanol nach Anspruch 1 zur Herstellung von Katalysatoren für die Polymerisierung von Olefinen.

## Claims

1. Storage-stable solutions of a mixture of carbonated magnesium methylate of the formula Mg(CH₃O)₂ (CO₂)ₙ, carbonated magnesium ethylate or the formula Mg(C₂H₅O)₂ (CO₂)ₙ and their carbonated mixed alkoxide of the formula Mg(CH₃O)ₓ (C₂H₅O)_{y} (CO₂)ₙ in a mixture of methanol and ethanol, where x and y are greater than 0 and less than 2 and the sum of x and y is equal to 2, characterized in that the magnesium content of the solution is 0.1 to 11% by weight, relative to the total solution, and the CO₂ content is n equal to 1.2 to 2.2.

2. Process for preparing storage-stable solutions of a mixture of carbonated magnesium methylate or the formula Mg(CH₃O)₂ (CO₂)ₙ, carbonated magnesium ethylate of the formula Mg(C₂H₅O)₂ (CO₂)ₙ and their carbonated mixed alkoxide of the formula Mg(CH₃O)ₓ (C₂H₅O)_{y} (CO₂)ₙ in a mixture of methanol and ethanol, where x and y are greater than 0 and less than 2 and the sum of x and y is equal to 2, according to Claim 1, characterized in that magnesium ethylate in methanol or magnesium ethylate and magnesium methylate in methanol or magnesium methylate in ethanol or magnesium methylate and magnesium ethylate in ethanol and/or their mixed alkoxide of the formula Mg(CH₃O)ₓ (C₂H₅O)_{y}, where x and y are greater than 0 and less than 2 and the sum of x and y is equal to 2, is/are reacted in ethanol or methanol with CO₂ and the magnesium content of the solution is adjusted to values of 0.1 to 11% by weight, relative to the total solution, and the CO₂ content is adjusted to values of n equal to 1.2 to 2.2.

3. Process for preparing storage-stable solutions of a mixture of carbonated magnesium methylate of the formula Mg(CH₃O)₂ (CO₂)ₙ, carbonated magnesium ethylate of the formula Mg(C₂H₅O)₂ (CO₂)ₙ and their carbonated mixed alkoxide of the formula Mg(CH₃O)ₓ (C₂H₅O)_{y} (CO₂)ₙ in a mixture of methanol and ethanol, where x and y are greater than 0 and less than 2 and the sum of x and y is equal to 2, according to claim 1, characterized in that the magnesium ethylate and/or magnesium methylate and/or their mixed alkoxide of the formula Mg(CH₃O)ₓ (C₃H₅O)_{y}, where x and y are greater than 0 and less than 2 and the sum of x and y is equal to 2, is/are reacted in a mixture of methanol and ethanol with CO₂ and the magnesium content of the solution is adjusted to values of 0.1 to 11% by weight, relative to the total solution, and the CO₂ content is adjusted to values of n equal to 1.2 to 2.2.

4. Process for preparing storage-stable solutions of a mixture of carbonated magnesium methylate of the formula Mg(CH₃O)₂ (CO₂)ₙ, carbonated magnesium ethylate of the formula Mg(C₂H₅O)₂ (CO₂)ₙ and their carbonated mixed alkoxide of the formula Mg(CH₃O)ₓ (C₂H₅O)_{y} (CO₂)ₙ in a mixture of methanol and ethanol, where x and y are greater than 0 and less than 2 and the sum of x and y is equal to 2, according to Claim 1, characterized in that metallic magnesium is reacted with a mixture of methanol and ethanol and of CO₂ and the magnesium content of the solution is adjusted to values of 0.1 to 11% by weight, relative to the total solution, and the CO₂ content is adjusted to values of n equal to 1.2 to 2.2.

5. Process according to Claims 2 to 4, characterized in that the CO₂ content n of the solution is adjusted by metered addition of gaseous, liquid or solid CO₂ to the mixture.

6. Process according to Claims 1 to 4, characterized in that the CO₂ content n of the solution is adjusted by thermally expelling CO₂ from a solution of a mixture of carbonated magnesium methylate, carbonated magnesium ethylate and their carbonated mixed alkoxide in a mixture of methanol and ethanol containing an excess of CO₂.

7. Process according to Claims 2 to 4, characterized in that the magnesium content of the solution is adjusted by distillative removal of methanol and/or ethanol from a solution of a mixture of carbonated magnesium methylate, carbonated magnesium ethylate and their carbonated mixed alkoxide in a mixture of methanol and ethanol containing an excess of methanol and/or ethanol.

8. Use of the storage-stable solutions of a mixture of carbonated magnesium methylate, carbonated magnesium ethylate and their carbonated mixed alkoxide in a mixture of methanol and ethanol according to Claim 1 for preserving paper.

9. Use of the storage-stable solutions of a mixture of carbonated magnesium methylate, carbonated magnesium ethylate and their carbonated mixed alkoxide in a mixture of methanol and ethanol according to Claim 1 for preparing catalysts for the polymerization of olefins.

## Revendications

1. Solutions stables au stockage d'un mélange de méthylate de magnésium carbonisé de la formule Mg(CH₃O)₂ (CO₂)ₙ, d'éthylate de magnésium carbonisé de la formule Mg(C₂H₅O)₂ (CO₂)ₙ et de leur alcoxyde mixte carbonisé de la formule Mg(CH₃O)ₓ (C₂H₅O)_{y} (CO₂)ₙ dans un mélange de méthanol et d'éthanol, dans lequel x et y sont plus grands que 0 et plus petits que 2 et la somme de x et de y est égale à 2,
caractérisées en ce que
la teneur en magnésium de la solution se monte à 0,1 jusqu'à 11 % en poids, par rapport à la solution totale, et la teneur en CO₂ n est égale à 1,2 jusqu'à 2,2.

2. Procédé de préparation de solutions stables au stockage d'un mélange de méthylate de magnésium carbonisé de la formule Mg(CH₃O)₂ (CO₂)ₙ, d'éthylate de magnésium carbonisé de la formule Mg(C₂H₅O)₂ (CO₂)ₙ et de leur alcoxyde mixte carbonisé de la formule Mg(CH₃O)ₓ (C₂H₅O)_{y} (CO₂)ₙ dans un mélange de méthanol et d'éthanol, dans lequel x et y sont supérieurs à 0 et inférieurs à 2 et la somme de x et y est égale à 2 selon la revendication 1,
caractérisé en ce que
de l'éthylate de magnésium dans du méthanol ou de l'éthylate de magnésium et du méthylate de magnésium dans du méthanol ou du méthylate de magnésium dans de l'éthanol ou du méthylate de magnésium et de l'éthylate de magnésium dans de l'éthanol et/ou leur alcoxyde mixte de la formule Mg(CH₃O)ₓ (C₂H₅O)_{y}, dans lequel x et y sont supérieurs à 0 et inférieurs à 2 et la somme de x et y est égale à 2, sont transformés dans de l'éthanol ou du méthanol avec CO₂ et la teneur en magnésium de la solution est ajustée à des valeurs de 0,1 à 11 % en poids, par rapport à la solution totale, et la teneur en CO₂ à des valeurs de n égales à 1,2 jusqu'à 2,2.

3. Procédé pour la préparation de solutions stables au stockage d'un mélange de méthylate de magnésium carbonisé de la formule Mg(CH₃O)₂ (CO₂)ₙ, d'éthylate de magnésium carbonisé de la formule Mg(C₂H₅O)₂ (CO₂)ₙ et de leur alcoxyde mixte carbonisé de la formule Mg(CH₃O)ₓ (C₂H₅O)_{y} (CO₂)ₙ dans un mélange de méthanol et d'éthanol, dans lequel x et y sont plus grands que 0 et plus petits que 2 et la somme de x et y est égale à 2, selon la revendication 1, caractérisé en ce que
l'éthylate de magnésium et/ou le méthylate de magnésium et/ou leur alcoxyde mixte de la formule Mg(CH₃O)ₓ (C₂H₅O)_{y} dans laquelle x et y sont plus grands que 0 et plus petits que 2 et la somme de x et y est égale à 2, sont transformés dans un mélange de méthanol et d'éthanol avec CO₂ et la teneur en magnésium de la solution est ajustée à des valeurs de 0,1 à 11 % en poids, par rapport à la solution totale, et la teneur en CO₂ à des valeurs de n égales à 1,2 jusqu'à 2,2.

4. Procédé pour la préparation de solutions stables au stockage d'un mélange de méthylate de magnésium carbonisé de la formule Mg(CH₃O)₂ (CO₂)ₙ, d'éthylate de magnésium carbonisé de la formule Mg(C₂H₅O)₂ (CO₂)ₙ et de leur alcoxyde mixte carbonisé de la formule Mg(CH₃O)ₓ (C₂H₅O)_{y} (CO₂)ₙ dans un mélange de méthanol et d'éthanol, dans lequel x et y sont plus grands que 0 et plus petits que 2 et la somme de x et y est égale à 2, selon la revendication 1, caractérisé en ce que
du magnésium métallique est transformé avec un mélange de méthanol et d'éthanol ainsi qu'avec du CO₂ et la teneur en magnésium de la solution est ajustée à des valeurs de 0,1 à 11 % en poids, par rapport à la solution totale, et la teneur en CO₂ à des valeurs de n égales à 1,2 jusqu'à 2,2.

5. Procédé selon les revendications 2 à 4,
caractérisé en ce que
la teneur en CO₂ n de la solution est ajustée par addition dosée de CO₂ gazeux, liquide ou solide dans le mélange.

6. Procédé selon les revendications 2 à 4,
caractérisé en ce que
la teneur en CO₂ n de la solution est ajustée par expulsion thermique de CO₂ d'une solution contenant un excédent de CO₂ d'un mélange de méthylate de magnésium carbonisé, d'éthylate de magnésium carbonisé et de leur alcoxyde mixte carbonisé dans un mélange de méthanol et d'éthanol.

7. Procédé selon les revendications 2 à 4,
caractérisé en ce que
la teneur en magnésium de la solution est ajustée par élimination distillative de méthanol et/ou d'éthanol d'une solution contenant un excédent de méthanol et/ou d'éthanol d'un mélange de méthylate de magnésium carbonisé, d'éthylate de magnésium carbonisé et de leur alcoxyde mixte carbonisé dans un mélange de méthanol et d'éthanol.

8. Utilisation des solutions stables au stockage d'un mélange de méthylate de magnésium carbonisé, d'éthylate de magnésium carbonisé et de leur alcoxyde mixte carbonisé dans un mélange de méthanol et d'éthanol selon la revendication 1 pour la conservation de papier.

9. Utilisation des solutions stables au stockage d'un mélange de méthylate de magnésium carbonisé, d'éthylate de magnésium carbonisé et de leur alcoxyde mixte carbonisé dans un mélange de méthanol et d'éthanol selon la revendication 1 pour la préparation de catalyseurs destinés à la polymérisation d'oléfines.
